# EUROPÄISCHE PATENTSCHRIFT

(11) **EP 2 256 183 B1**
(45) Veröffentlichungstag und Bekanntmachung des Hinweises auf die Patenterteilung: **08.05.2013**
(21) Anmeldenummer: 10075232.8
(22) Anmeldetag: 28.05.2010
(51) Int. Cl.: C12M 1/00, C12N 5/00

(54) **Kultivierungsstruktur, Verfahren zur Herstellung sowie Kultivierungsverfahren für phototrophe Mikroorganismen und diesbezügliche Verwendung eines Tensids**
Cultivation structure, method for manufacturing and cultivating phototrophic micro organisms and related use of a tenside
Structure de culture, procédé de fabrication et procédé de culture pour microorganismes phototrophes et utilisation d'un tensioactif correspondant

(30) Priorität: 29.05.2009 DE 102009026614
(43) Veröffentlichungstag der Anmeldung: 01.12.2010
(73) Patentinhaber: IGV Institut für Getreideverarbeitung GmbH, 14558 Bergholz-Rehbrücke (DE)
(72) Erfinder: Broneske, Jürgen, 14947 Nuthe-Urstromtal (DE); Pulz, Otto, 14558 Nuthetal (DE); Rothe, Thomas, 14558 Nuthetal (DE); Schmidt, Karsten, 16348 Wandlitz (DE); Sandau, Ekkehardt, 14558 Nuthetal (DE)
(74) Vertreter: Brandt, Detlef

(56) Entgegenhaltungen:
- DE-C1- 4 411 486
- JP-A- 11 181 678
- JP-A- 63 196 282
- US-A1- 2009 029 445

## Beschreibung

Die Erfindung bezieht sich auf die Kultivierung phototropher Mikroorganismen. Sie bezieht sich auf eine dafür zu verwendende Kultivierungsstruktur in Form eines Abschnitts einer gewebeartigen Materialbahn und auf ein Verfahren zu deren Herstellung. Ferner betrifft die Erfindung ein Verfahren zur Kultivierung der Mikroorganismen, welches insbesondere unter Verwendung der erfindungsgemäßen sowie erfindungsgemäß hergestellten Kultivierungsstruktur durchführbar ist. Sie bezieht sich außerdem auf die diesbezügliche Verwendung eines anionischen Tensids.

Zur Kultivierung phototropher Mikroorganismen ist es bekannt, entsprechende in einer Nährlösung enthaltene Mikroorganismen, wie beispielsweise Mikroalgen, an einer großflächigen, gewebeartigen Kultivierungsstruktur beziehungsweise an mehreren Kultivierungsstrukturen zu immobilisieren, das Wachstum der an den Kultivierungsstrukturen immobilisierten Mikroorganismen durch Lichteintrag und gegebenenfalls Zuführung weiterer, im Einzelfalle auch gasförmiger Nährstoffe zu unterstützen und sie schließlich mittels unterschiedlicher physikalischer Verfahren, wie beispielsweise mechanische Verfahren, von den Kultivierungsstrukturen abzuernten. Dazu werden die entsprechenden Kultivierungsstrukturen gemäß gebräuchlicher Verfahren in vertikaler oder zumindest überwiegend vertikaler Lage angeordnet und die mit den Mikroorganismen inokulierte Nährlösung oberhalb der Kultivierungsstrukturen ausgebracht. Ein derartiges Verfahren ist zum Beispiel in der DE 44 11 486 C1 offenbart.

Damit sich die Mikroorganismen an den Kultivierungsstrukturen gut anlagern können, müssen deren Oberflächen hydrophil sein beziehungsweise durch die Nährlösung gut durchfeuchtet werden können. Im Hinblick auf einen guten Lichteintrag in größere, mit mehreren parallel zueinander angeordneten Kultivierungsstrukturen ausgestattete Kultivierungsanlagen werden die Kultivierungsstrukturen zudem möglichst dünn ausgebildet. In der Praxis werden zu diesem Zweck im Allgemeinen hydrophile, vliesartige Gewebe aus Kunst-und/oder Naturfasern verwendet, welche aufgrund ihrer geringen Dicke lichtdurchlässig ausgebildet sind.

Allerdings hat es sich gezeigt, dass hierbei insbesondere bei einer Kultivierung größerer Mengen phototropher Mikroorganismen Probleme auftreten. Die Kultivierungsstrukturen werden nämlich durch die sich anlagernde Menge von Mikroorganismen häufig so schwer, dass ihre mechanische Stabilität sich deutlich verringert. Dies ist zumindest im Hinblick auf den sich an das Wachstum der Mikroorganismen anschließenden, häufig mechanischen Erntevorgang problematisch, da die Gefahr besteht, dass die Kultivierungsstrukturen bei der Ernte der an ihnen immobilisierten Mikroorganismen reißen oder in sonstiger Weise beschädigt beziehungsweise zerstört werden. Dabei nimmt diese Gefahr bei längerer beziehungsweise wiederholter Nutzung der Kultivierungsstrukturen stark zu.

Aufgrund dieser mangelnden Langzeitstabilität sind entsprechende Kultivierungsstrukturen zumindest für eine Massenproduktion von Mikroorganismen, bei welcher eine Vielzahl sehr großflächiger Kultivierungsstrukturen zum Einsatz gelangt, im Grunde ungeeignet, da ein ständiger Ersatz verschlissener Kultivierungsstrukturen aufwendig und teuer sowie einer weitgehend unterbrechungsfreien Produktion abträglich wäre. Dem gegenüber stabiler sind Folien oder Gewebe aus hydrophoben Materialen, welche aber andererseits, aus den schon erläuterten Gründen, nicht ohne weiteres zur Immobilisierung der Mikroorganismen geeignet sind. Daher sind Lösungen vorgeschlagen worden, nach welchen als Kultivierungsstrukturen mehrschichtige Laminate mit einem hydrophoben Kern und einer hydrophilen Oberfläche beziehungsweise mit entsprechenden hydrophilen Materialen beschichtete hydrophobe Folienmaterialien beziehungsweise Gewebe verwendet werden. So wird beispielsweise gemäß der JP 63 19 62 82 A zur Kultivierung eine Kultivierungsstruktur verwendet, bei der ein polymeres Basismaterial, wie Polyester, mit einem Multilayer monomolekularer Filme beschichtet wird. Dabei wird durch das Basismaterial, aus dem als solches hydrophoben Polyester eine gute Stabilität der Kultivierungsstruktur erreicht, während die mit den monomolekularen Filmen beschichtete Oberfläche das Anlagern der Mikroorganismen begünstigt. Zur Beschichtung des Basismaterials werden die entsprechenden Filmmaterialien aufgetragen und unter Einwirkung von UV-Licht oder einer Ionenbestrahlung in Form eines Gitters, von Streifen oder von Punkten haftend mit dem Basismaterial verbunden. Eine andere Möglichkeit besteht gemäß der JP 10 2003 00 26 943 A darin, ein hydrophobes Material hinreichender Stabilität, wie Polyolephin, mittels einer Plasma-Ionen-Behandlung so zu verändern, dass entsprechende Mikroorganismen an seiner Oberfläche immobilisiert werden können. Die beiden vorgenannten Verfahren beziehungsweise die entsprechenden mehrschichtigen Kultivierungsstrukturen sind jedoch aufgrund der dabei bestehenden technologischen Erfordernisse verhältnismäßig teuer. Dies ist aber im Hinblick auf eine Massenproduktion von Mikroorganismen und die dazu benötigten großen Kultivierungsflächen ebenfalls nachteilig.

Aufgabe der Erfindung ist es daher, eine alternative Lösung anzugeben, welche die vorstehend genannten Probleme löst und eine großflächige Massenproduktion von phototrophen Mikroorganismen begünstigt. Dazu ist eine Kultivierungsstruktur zu schaffen, welche eine Anlagerung hinreichend großer Mengen von Mikroorganismen ermöglicht und auch im Falle einer großflächigen Auslegung grundsätzlich die erforderliche Stabilität sowie eine gute Langzeitstabilität besitzt. Ferner sind ein Verfahren zur Herstellung einer derartigen Kultivierungsstruktur anzugeben und ein insbesondere unter Verwendung der erfindungsgemäßen Kultivierungsstruktur durchführbares Kultivierungsverfahren sowie die diesbezügliche Verwendung eines anionischen Tensids zu beschreiben.

Die Aufgabe wird durch eine Kultivierungsstruktur mit den Merkmalen des Hauptanspruchs gelöst. Ein die Aufgabe lösendes Verfahren zur Herstellung der entsprechenden Kultivierungsstruktur ist durch den ersten verfahrensbezogenen Anspruch charakterisiert. Ferner ist durch den ersten, sich auf die Kultivierung beziehenden Anspruch ein die Aufgabe lösendes Kultivierungsverfahren charakterisiert. Vorteilhafte Aus- beziehungsweise Weiterbildungen der Erfindung sind durch die jeweiligen Unteransprüche gegeben.

Bei der erfindungsgemäßen, zur Verwendung bei der Kultivierung phototropher Mikroorganismen vorgesehenen Kultivierungsstruktur handelt es sich um den Abschnitt einer gewebeartigen Materialbahn, welche foliengleich dünn ausgebildet ist. Mindestens eine der beiden großen, aufgrund der flachen gewebeartigen Beschaffenheit strukturierten Oberflächen dieses Abschnitts der Materialbahn bildet bei der Kultivierung der Mikroorganismen eine Immobilisierungsfläche für die in einer Nährlösung über die betreffende Oberfläche verteilten Mikroorganismen aus. Erfindungsgemäß besteht die Kultivierungsstruktur aus einem zumindest überwiegend hydrophoben Material, wobei aber ihre Immobilisierungsfläche oder -flächen infolge einer wenigstens einmaligen temporären Benetzung mit einem Netzmittel eine dauerhaft hydrophile Struktur aufweist beziehungsweise aufweisen.

An dieser Stelle ist hervorzuheben, dass das Material der erfindungsgemäßen Kultivierungsstruktur hydrophob beziehungsweise zumindest aber überwiegend hydrophob ist, dieses jedoch zur Ausbildung der Immobilisierungsfläche oder -flächen, anders als aus dem Stand der Technik bekannt, nicht mit einer zusätzlichen hydrophilen Beschichtung versehen ist. Vielmehr resultiert der hydrophile Charakter der mindestens einen Immobilisierungsfläche der Kultivierungsstruktur aus der zumindest temporären einmaligen Benetzung der entsprechenden Oberfläche mit einem Netzmittel, wobei dies, wie noch ausgeführt werden soll, einer dauerhaften beziehungsweise fortwährenden Benetzung der betreffenden Oberfläche mit dem Netzmittel nicht entgegensteht.
Überraschenderweise hat sich gezeigt, dass eine einmalige temporäre Benetzung der zur Immobilisierung dienenden Oberfläche des hydrophoben Materials mit einem geeigneten Netzmittel ausreichend ist, um der Oberfläche einen dauerhaft hydrophilen Charakter zu verleihen. Die dies bewirkenden Mechanismen sind jedoch noch nicht vollständig bekannt. Festzustellen beziehungsweise nochmals zu betonen ist jedoch, dass keine Beschichtung des Materials erfolgt. Zudem ist davon auszugehen, dass es zwischen dem hydrophoben Material und dem Netzmittel zu keiner chemischen Reaktion kommt. Es wird daher angenommen, dass die dauerhafte Veränderung der Oberfläche, durch welche ihr ein hydrophiler Charakter verliehen wird, der auch bestehen bleibt, sofern eine weitere Benetzung später nicht mehr erfolgt, durch eine Veränderung der Oberflächenstruktur bewirkt wird. Die Oberfläche wird dabei aber anders aussehen beziehungsweise beschaffen sein, als etwa nach einer Plasma-Ionen-Behandlung.

Gemäß einer bevorzugten Ausbildungsform der Erfindung besteht die Kultivierungsstruktur aus einem Gewebe, dessen Bestandteil Fasern mindestens eines hydrophoben Polymers sind. Bei entsprechenden Versuchen haben sich insoweit insbesondere Gewebe aus Polyesterfasern oder aus Polyethylenfasern als vorteilhaft erwiesen. Auch Gewebe aus Polypropylenfasern kommen in Betracht. Ferner ist die Verwendung von Mischgeweben, welche aus mehreren der vorgenannten Materialien bestehen, denkbar. Darüber hinaus hat sich in Versuchen die Verwendung von Geweben aus einem Fasermix mit Fasern eines oder mehrerer der vorgenannten hydrophoben Polymere und künstlichen Fasern aus Naturstoffen, wie zum Beispiel Viskose, als praktikabel erwiesen. Da erfindungsgemäß im Hinblick auf die geforderte Stabilität beziehungsweise insbesondere Langzeitstabilität der Kultivierungsstrukturen für diese ein überwiegend aber nicht zwingend vollständig hydrophobes Material verwendet wird, umfasst die Erfindung grundsätzlich auch die Möglichkeit, in einem Fasermix zur Herstellung des Gewebes einen vorzugsweise geringen Anteil hydrophiler Naturfasern zu verwenden. Insbesondere kommt hierbei die Zugabe zellulosebasierter Fasern in Betracht. Derartige, auf einem Fasermix basierende Kultivierungsstrukturen könnten sich insbesondere im Hinblick auf eine bessere Umweltverträglichkeit als ökologisch vorteilhaft erweisen.

Es sei aber nochmals betont, dass das Material der Kultivierungsstruktur insbesondere zur Erreichung der angestrebten Langzeitstabilität in jedem Falle überwiegend aus als solches hydrophoben Materialien besteht. Das heißt, dass der Anteil hydrophober Materialien in einem entsprechenden Gewebe über 50 % beträgt, so dass dieses ohne die Veränderung seiner Oberflächenstruktur durch eine mindestens einmalige Benetzung mit dem Netzmittel im Grunde nicht zu Kultivierungszwecken für phototrophe Mikroorganismen geeignet ist.

Um den bei der Kultivierung phototropher Mikroorganismen erforderlichen Lichteintrag zu begünstigen, ist die erfindungsgemäße Kultivierungsstruktur zudem, gemäß einer bevorzugten Ausbildungsform, lichtdurchlässig ausgebildet, was sich durch eine entsprechende Materialwahl und -stärke der das Gewebe ausbildenden Fasern bewerkstelligen lässt. Je nach dem Lichtregime und der Art des Lichteinfalls in die Kultivierungsanlage kann die Kultivierungsstruktur aber auch so ausgebildet sein, dass einfallendes Licht von ihrer Oberfläche gestreut, das heißt an der Mikrostruktur der Oberfläche durch gewissermaßen partielle Reflektion verteilt wird oder von dieser in das Materialinnere geleitet wird. Ziel ist es in jedem Falle die Kultivierungsstrukturen so auszubilden, dass sichergestellt ist, dass auch bei einer Vielzahl, vorzugsweise mit zumindest überwiegend vertikaler Orientierung parallel hängender Kultivierungsstrukturen deren Oberflächen durch das Licht möglichst gleichmäßig ausgeleuchtet werden.

Nach dem erfindungsgemäß, zur Lösung der gestellten Aufgabe vorgeschlagenen Verfahren zur Erzeugung einer Kultivierungsstruktur für die Kultivierung phototropher Mikroorganismen wird auf mindestens einer der großen Oberflächen eines Abschnitts einer gewebeartigen Materialbahn eine Immobilisierungsfläche erzeugt. Die entsprechende Oberfläche beziehungsweise Oberflächen dienen der Immobilisierung in einer Nährlösung enthaltener und zum Zwecke ihrer Kultivierung über die jeweilige Oberfläche verteilter Mikroorganismen. Erfindungsgemäß wird zur Herstellung der Kultivierungsstruktur eine Materialbahn aus einem zumindest überwiegend hydrophoben Material verwendet. Die zur Ausbildung einer Immobilisierungsfläche vorgesehene Oberfläche oder vorgesehenen Oberflächen der betreffenden Materialbahn wird beziehungsweise werden mit einer Lösung benetzt, welche wenigstens ein Tensid in einer Konzentration zwischen 10 ppm und 50 ppm enthält. Ferner werden mindestens ein der vorgesehenen Geometrie der Kultivierungsstruktur entsprechender Abschnitt der betreffenden Materialbahn konfektioniert, das heißt insbesondere auf Maß geschnitten und an diesem gegebenenfalls Ösen Schlaufen oder dergleichen angebracht oder erzeugt, welche es zum Beispiel ermöglichen, die betreffende Kultivierungsstruktur in einer Kultivierungsanlage aufzuhängen. Je nach Ausgestaltung des erfindungsgemäßen Verfahrens können die beiden vorgenannten Verfahrensschritte, also das Benetzen der Materialbahn mit dem wenigstens einen Tensid einerseits und deren Konfektionierung entsprechend der Geometrie der herzustellenden Kultivierungsstruktur andererseits, auch in umgekehrter Reihenfolge durchgeführt werden. Die zur Benetzung der hydrophoben Materialbahn verwendete Lösung enthält das wenigstens eine Tensid gemäß einer bevorzugten Verfahrensführung in einer Konzentration von etwa 20 ppm. Im Hinblick auf die schon angesprochene mögliche unterschiedliche Reihenfolge der vorgenannten Verfahrensschritte ist eine praktikable Verfahrensgestaltung dadurch gegeben, dass der letztlich die Kultivierungsstruktur für die Kultivierung ausbildende Abschnitt einer entsprechenden Materialbahn erst nach der Benetzung einer oder beider seiner großen Oberflächen mit der tensidhaltigen Lösung entsprechend der Geometrie der zu erzeugenden Kultivierungsstruktur konfektioniert wird. Die Erzeugung der Immobilisierungsfläche oder der Immobilisierungsflächen erfolgt dabei im Zuge der Herstellung der Materialbahn vorzugsweise durch ein Einsprühen der Materialbahn mit der entsprechenden Lösung. Denkbar ist aber auch ein Tauchvorgang, bei welchem die Materialbahn beispielsweise von einem Wickel auf einen anderen umgewickelt und dabei durch ein entsprechendes Tauchbad geführt wird. Im Hinblick auf eine entsprechend große Fertigungskapazität für die Kultivierungsstrukturen scheint es dabei vorteilhaft möglich, dass ein sehr kurzzeitiges, im Zuge eines schnellen Durchlaufs der Materialbahn durch das Tauchbad erfolgendes Benetzen der Oberfläche oder Oberflächen ausreichend ist, um die gewünschte Veränderung der Oberflächenstruktur herbeizuführen.

Gemäß einer grundsätzlich anderen Verfahrensführung erfolgt die Benetzung der Oberflächen der Kultivierungsstruktur mit der das wenigstens eine Tensid enthaltenden Lösung erst nach dem Zuschnitt beziehungsweise der Konfektionierung eines jeweiligen Abschnitts der Materialbahn. Die Benetzung der zur Immobilisierung der Mikroorganismen vorgesehenen Oberflächen kann hierbei vorteilhafterweise auch erst bei der Nutzung der Kultivierungsstruktur für die Kultivierung erfolgen, und zwar beim Benetzen der Kultivierungsstrukturen mit der mit den phototrophen Mikroorganismen zu inokulierenden Nährlösung. Hierfür wird der Nährlösung wenigstens ein Tensid in der bereits angegebenen Konzentration beigegeben. Vorzugsweise handelt es sich bei dem wenigstens einen Tensid um ein anionisches Tensid, wobei sich insbesondere die Verwendung von Alkylbenzinsulfonat als besonders wirkungsvoll erwiesen hat.

Nach dem vorgeschlagenen Kultivierungsverfahren für phototrophe Mikroorganismen werden die Mikroorganismen, wie grundsätzlich bereits bekannt, an mindestens einer, im Wesentlichen vertikal angeordneten Kultivierungsstruktur aus einem zumindest überwiegen hydrophoben Material immobilisiert. Hierzu wird oberhalb der mindestens einen Kultivierungsstruktur eine die Mikroorganismen enthaltende Nährlösung ausgebracht. Die an der oder den entsprechenden Oberflächen der Kultivierungsstruktur immobilisierten Mikroorganismen werden schließlich unter Zufuhr von natürlichem oder künstlichem Licht sowie gasförmigen Nährstoffen an der Kultivierungsstruktur in bekannter Weise kultiviert. Erfindungsgemäß ist das Kultivierungsverfahren jedoch abweichend vom Stand der Technik so gestaltet, dass die ausgebrachte Nährlösung mindestens ein Tensid enthält, wobei die Konzentration des mindestens einen Tensids beim erstmaligen Ausbringen der in einem Kreislauf geführten Nährlösung zwischen 10 ppm und 50 ppm beträgt. Zwar zeigt sich bei dieser Verfahrensweise eine leichte, im Grunde unerwünschte Schaumbildung in der Nährlösung, jedoch kann dies beherrscht werden. Wie gefunden wurde, wirkt sich die Zugabe des Tensids insoweit weder unter dem vorgenannten Gesichtspunkt, noch im Hinblick auf einen möglichen Einfluss des Tensids auf das Wachstum der Mikroorganismen nachteilig aus.

Ein unter Verwendung der erfindungsgemäß ausgebildeten und hergestellten Kultivierungsstruktur durchgeführtes Kultivierungsverfahren für phototrophe Mikroorganismen zeichnet sich dadurch aus, dass Tensidanteile, welche während des zeitlich länger andauernden Kultivierungsprozesses durch die in einem ständigen Umlauf geführte Nährlösung von den mit dem Netzmittel behandelten Immobilisierungsflächen wieder herunter gewaschen werden, in der Nährlösung verbleiben. Gleiches gilt im Falle einer Erzeugung der Immobilisierungsflächen durch Benetzung mit einer das oder die Tenside enthaltenden und mit Mikroorganismen zu inokulierenden Nährlösung für dabei gegebenenfalls überschüssige Tensidanteile. Das heißt, es ist insoweit nicht erforderlich, das oder die Tenside durch aufwendige Maßnahmen wieder aus der Nährlösung herauszufiltern. Überraschenderweise und anders als zu erwarten, hat sich gezeigt, dass die verwendeten Tenside auf die Mikroorganismen nicht toxisch wirken und deren Wachstum nicht behindern. Es ist vielmehr sogar festzustellen, dass das Wachstum der Mikroorganismen durch die verwendeten Tenside begünstigt wird, da eine Kontamination des Kultivierungssystems mit Fremdorganismen, wie insbesondere Pilzen oder Bakterien durch das Wirken der Tenside offenbar zumindest teilweise unterbunden wird. Das erfindungsgemäße Kultivierungsverfahren lässt sich mit besonderem Vorteil insbesondere zur Kultivierung von Mikroalgen nutzen. Es hat sich in Versuchen als besonders vorteilhaft für die Kultivierung von Mikroalgen der Spezies Chlorella vulgaris erwiesen.

Grundzüge der Erfindung sollen nachfolgend nochmals in der Art eines Ausführungsbeispiels näher erläutert werden. Das Beispiel bezieht sich auf eine Verfahrensvariante zur Herstellung erfindungsgemäßer Kultivierungsstrukturen, bei welcher die Herstellung im Zuge der Inbetriebnahme einer neuen oder neu mit entsprechenden Kultivierungsstrukturen auszustattenden Kultivierungseinrichtung erfolgt. Dabei wird von einer Einrichtung ausgegangen, welche in Form eines Trenchsystems ausgebildet ist. In der Kultivierungseinrichtung werden hierzu mehrere, noch nicht final behandelte Kultivierungsstrukturen in vorzugsweise paralleler und im Wesentlichen vertikaler Anordnung aufgehängt. Das Trenchsystem umfasst Behälter, Rohrleitungen, wenigstens eine Pumpe, mehrere so genannte Trenche sowie Ventile und Mittel, beispielsweise Tropfschläuche, zur Ausbringung einer die Mikroorganismen enthaltenden Nährlösung. Klarstellend sei an dieser Stelle nochmals bemerkt, dass die Erzeugung der Kultivierungsstrukturen für die Immobilisierung der Mikroalgen zu diesem Zeitpunkt, das heißt vor Inbetriebnahme der Kultivierungseinrichtung, im Grunde noch nicht abgeschlossen ist, da diese sich zunächst lediglich als Abschnitte einer hydrophoben Materialbahn darstellen. Dennoch soll in diesem Zusammenhang, zur Vereinfachung, bereits von Kultivierungsstrukturen gesprochen werden, auch wenn die entsprechenden Abschnitte der Materialbahn erst nach einer wenigstens einmaligen Benetzung mit der das oder die Tenside enthaltenden Lösung zu Kultivierungsstrukturen, im Sinne mit mindestens einer Immobilisierungsfläche ausgestatteter Träger, für die zu kultivierenden Mikroorganismen werden.

Oberhalb der Kultivierungsstrukturen sind die bereits erwähnten Tropfschläuche angeordnet, deren Auslassöffnungen für die Nährlösung so platziert sind, dass die austretende Nährlösung beim späteren Betrieb der Anlage unmittelbar auf die Kultivierungsstrukturen herunterfällt. Bei den ebenfalls schon angesprochenen Trenchen handelt es sich um Sammelrinnen, in welche die überschüssige, nicht von den Kultivierungsstrukturen aufgenommene Nährlösung abläuft. Die Nährlösung wird in der Anlage in einem ständigen Umlauf bewegt, welchem sie über die Trenche wieder zugeführt wird.

Es sei angenommen, dass die Kultivierungsanlage mit etwa 200.000 I Wasser betrieben wird. Die entsprechende Wassermenge wird zunächst ohne eine Benetzung der Kultivierungsstrukturen in der Anlage mittels einer Pumpe in Umlauf gebracht. Dazu sind in der Anlage entsprechende Rohrleitungswege vorgesehen und darüber hinaus vorhandene Ventile so eingestellt, dass ein entsprechender Kreislauf unter Umgehung der Kultivierungsstrukturen gebildet ist. Die zur Förderung des Wassers beziehungsweise der späteren wässrigen Lösung dienende Trenchpumpe wird zunächst auf einen minimalen Durchsatz eingestellt. Nun werden etwa 8 kg eines Tensids, vorzugsweise linearen Alkylbenzinsulfonats, mit warmem Wasser verdünnt beziehungsweise aufgelöst und dann schrittweise dem in Umlauf befindlichen Wasser zudosiert. Hierbei ist, wie von Tensiden allgemein bekannt, eine gewisse Schaumbildung zu verzeichnen, welche jedoch durch den eingestellten minimalen Durchsatz für die in Umlauf gehaltene Flüssigkeit möglichst gering gehalten wird. Nach der Zugabe des LAS (des gelösten Alkylbenzinsulfonats - Linear Alkylate Sulfonate Solution) werden das Netzmittel und das Wasser durch Aufrechterhaltung des Umlaufs für eine Dauer von ca. 2 bis 3 Stunden vollständig miteinander vermischt, so dass eine gewisse Homogenisierung eintritt. Danach wird die Anlage schrittweise hochgefahren. Das heißt, es werden je nach konstruktiver Beschaffenheit schrittweise einzelne Gruppen der Kultivierungsstrukturen in den Umlauf mit einbezogen und der Durchsatz der Anlage mit der Nährlösung erhöht. Es hat sich gezeigt, dass der Grad der Benetzung der gewebe- beziehungsweise vliesartigen Kultivierungsstrukturen temperaturabhängig ist, wobei die Benetzung durch höhere Temperaturen begünstigt wird. Daher erfolgt die Benetzung vorzugsweise unter Nutzung von die Anlage umgebendem Sonnenlicht. Da die Randbereiche der Kultivierungsstrukturen im Hinblick auf die Benetzung etwas kritisch sind, kann es vorteilhaft sein, diese mittels eines Hochdrucksystems zusätzlich zu benetzen. Schließlich werden der im Umlauf befindlichen Flüssigkeit die zur Kultivierung der Mikroorganismen erforderlichen Nährstoffe zugesetzt und dann die Kultivierung durch Inokulation der so erhaltenen Nährlösung mit den phototrophen Mikroorganismen, vorzugsweise mit Mikroalgen der Spezies Chlorella vulgaris in Gang gesetzt.

Zur Förderung des Wachstums der Biomasse werden der Anlage nun außerdem CO₂ zugegeben und gegebenenfalls die Lichtzufuhr verstärkt beziehungsweise entsprechend bekannter Kultivierungsregimes zeitlich gesteuert und geregelt. Die solchermaßen kultivierte Biomasse wird schließlich bei Erreichung eines festgelegten Grades der Bedeckung der Kultivierungsstrukturen mit der Biomasse mittels bekannter mechanischer Ernteverfahren wieder von den Kultivierungsstrukturen entfernt und der gegebenenfalls weiteren Verarbeitung zugeführt.

## Patentansprüche

1. Kultivierungsstruktur zur Verwendung bei der Kultivierung phototropher Mikroorganismen, in Form eines Abschnitts einer gewebeartigen Materialbahn, wobei mindestes eine der beiden großen Oberflächen der Kultivierungsstruktur bei der Kultivierung eine Immobilisierungsfläche für in einer Nährlösung über die betreffende Oberfläche verteilte Mikroorganismen ausbildet, **dadurch gekennzeichnet, dass** die Kultivierungsstruktur aus einem zumindest überwiegend hydrophoben Material besteht, aber ihre Immobilisierungsfläche oder -flächen in Folge einer wenigstens einmalig temporären Benetzung mit einem Netzmittel eine dauerhaft hydrophile Struktur aufweisen.

2. Kultivierungsstruktur nach Anspruch 1, **dadurch gekennzeichnet, dass** diese aus einem Gewebe besteht, dessen Bestandteil Fasern mindestens eines hydrophoben Polymers sind.

3. Kultivierungsstruktur nach Anspruch 2, **dadurch gekennzeichnet, dass** das Gewebe aus einem Fasermix mit Fasern eines oder mehrerer hydrophober Polymere und Naturfasern besteht.

4. Kultivierungsstruktur nach einem der Ansprüche 1 bis 3, **dadurch gekennzeichnet, dass** die Kultivierungsstruktur lichtdurchlässig ist.

5. Kultivierungsstruktur nach einem der Ansprüche 1 bis 3, **dadurch gekennzeichnet, dass** die Kultivierungsstruktur mindestens eine einfallendes Licht streuende Immobilisierungsfläche besitzt.

6. Kultivierungsstruktur nach einem der Ansprüche 1 bis 3, **dadurch gekennzeichnet, dass** die Kultivierungsstruktur lichtleitend ausgebildet ist, wobei mindestens auf eine Immobilisierungsfläche auftreffendes Licht zumindest teilweise in das Materialinnere geleitet wird.

7. Verfahren zur Erzeugung einer Kultivierungsstruktur zur Kultivierung phototropher Mikroorganismen, bei welchem auf mindestens einer der großen Oberflächen eines Abschnitts einer gewebeartigen Materialbahn eine Immobilisierungsfläche für in einer Nährlösung zum Zwecke ihrer Kultivierung über die betreffende Oberfläche verteilte Mikroorganismen erzeugt wird, **dadurch gekennzeichnet, dass** zur Herstellung der Kultivierungsstruktur eine Materialbahn aus einem zumindest überwiegend hydrophoben Material verwendet wird und
a.) die zur Ausbildung einer Immobilisierungsfläche vorgesehene Oberfläche oder vorgesehenen Oberflächen der betreffenden Materialbahn mit einer Lösung benetzt wird oder werden, welche wenigstens ein Tensid in einer Konzentration zwischen 10 ppm und 50 ppm enthält,
b.) mindestens ein Abschnitt entsprechend der für die Kultivierungsstruktur vorgesehenen Geometrie konfektioniert wird,
wobei die beiden vorgenannten Schritte a.) und b.) auch in umgekehrter Reihenfolge durchgeführt werden können.

8. Verfahren nach Anspruch 7, **dadurch gekennzeichnet, dass** zur Benetzung der Oberfläche oder Oberflächen der hydrophoben Materialbahn eine Lösung verwendet wird, welche wenigstens ein Tensid in einer Konzentration von etwa 20 ppm enthält.

9. Verfahren nach Anspruch 7 oder 8, wobei nach der Benetzung der oder beider großer Oberflächen der Materialbahn mit der tensidhaltigen Lösung mindesten ein Abschnitt entsprechend der Geometrie der auszubildenden Kultivierungsstruktur konfektioniert wird, **dadurch gekennzeichnet, dass** die Erzeugung der Immobilisierungsfläche oder -flächen im Zuge der Herstellung der Materialbahn durch Einsprühen der Materialbahn mit wenigstens einem Tensid erfolgt.

10. Verfahren nach Anspruch 7 oder 8, wobei nach der Benetzung der oder beider großer Oberflächen der Materialbahn mit der tensidhaltigen Lösung mindesten ein Abschnitt entsprechend der Geometrie der auszubildenden Kultivierungsstruktur konfektioniert wird, **dadurch gekennzeichnet, dass** die Erzeugung der Immobilisierungsfläche oder -flächen im Zuge der Herstellung der Materialbahn durch Aufbringung wenigstens eines Tensids in einem Tauchverfahren erfolgt, bei welchem die jeweils zu benetzenden Abschnitte der Materialbahn in die tensidhaltige Lösung eingetaucht werden.

11. Verfahren nach Anspruch 7 oder 8, wobei ein entsprechender Abschnitt für die Kultivierungsstruktur ausgehend von der Materialbahn des hydrophoben Materials vor der Benetzung der oder beider großer Oberflächen der Materialbahn mit der tensidhaltigen Lösung entsprechend der Geometrie Kultivierungsstruktur konfektioniert wird, **dadurch gekennzeichnet, dass** die Benetzung mit der tensidhaltigen Lösung bei der Nutzung der Kultivierungsstruktur für die Kultivierung erfolgt, wobei das mindestens eine Tensid einer mit den phototrophen Mikroorganismen bereits inokulierten oder zu inokulierenden sowie für deren Kultivierung mit Nährstoffen anzureichernden und zum Zwecke der Immobilisierung der Mikroorganismen auf die Kultivierungsstruktur aufzubringenden Lösung beigegeben wird.

12. Verfahren nach Anspruch 11, **dadurch gekennzeichnet, dass** der oder die als Kultivierungsstruktur für die Kultivierung von Mikroorganismen dienenden Abschnitte einer Materialbahn hydrophoben Materials zur Ausbildung mindestens einer Immobilisierungsfläche bei der Inbetriebnahme der Kultivierungseinrichtung oder der ersten Ingebrauchnahme als Kultivierungsstruktur intensiv mit der tensidhaltigen Lösung benetzt werden, wozu eine mit den Mikroorganismen zu inokulierende wässrige Lösung mit einem gegenüber dem eigentlichen Kultivierungsbetrieb verringerten Durchsatz zunächst ohne eine Benetzung der Kultivierungsstrukturen in einem wiederholten Umlauf durch die Kultivierungseinrichtung geführt, dieser dabei stufenweise das oder die Tenside zudosiert werden und die Lösung nach der Durchmischung mit dem oder den Tensiden unter Zugabe von Nährstoffen und schrittweiser Erhöhung des Durchsatzes der Nährlösung auf die dann in den Umlauf mit einbezogenen Kultivierungsstrukturen ausgebracht wird.

13. Verfahren nach einem der Ansprüche 7 bis 12, **dadurch gekennzeichnet, dass** zur Benetzung der als Immobilisierungsflächen dienenden Oberflächen der Kultivierungsstruktur ein anionisches Tensid verwendet wird.

14. Verfahren nach Anspruch 13, **dadurch gekennzeichnet, dass** zur Benetzung lineares Alkylbenzensulfonat zur Benetzung verwendet wird.

15. Kultivierungsverfahren für phototrophe Mikroorganismen, nach welchem die Mikroorganismen an mindestens einer im wesentlichen vertikal angeordneten Kultivierungsstruktur aus einem zumindest uberwiegend hydrophoben Material immobilisiert werden, wobei oberhalb der mindestens einen Kultivierungsstruktur eine wässrige mit den Mikroorganismen zu inokulierende Nährlösung ausgebracht und die nach der Inokulation an der mindestens einen Kultivierungsstruktur immobilisierten Mikroorganismen unter Zufuhr von natürlichem oder künstlichem Licht sowie gasförmigen Nährstoffen kultiviert werden, **dadurch gekennzeichnet, dass** die oberhalb der mindestens einen Kultivierungsstruktur ausgebrachte Nährlösung mindestens ein Tensid enthält, wobei die Konzentration des mindestens einen Tensids beim erstmaligen Ausbringen der in einem Kreislauf geführten Nährlösung zwischen 10 ppm und 50 ppm beträgt.

16. Kultivierungsverfahren für phototrophe Mikroorganismen unter Verwendung mindestens einer Kultivierungsstruktur nach Anspruch 1, welches nach dem Verfahren gemäß einem der Ansprüche 7 bis 14 hergestellt wurde, wobei oberhalb der mindestens einen Kultivierungsstruktur eine Nährlösung mit den darin enthaltenen Mikroorganismen ausgebracht wird, **dadurch gekennzeichnet, dass** Tensidanteile, welche wieder von der oder den Immobilisierungsflächen herunter gewaschen werden oder als überschüssige Tensidanteile in der Nährlösung enthalten sind, für die Dauer des Kultivierungsvorgangs in der Nährlösung verbleiben.

17. Kultivierungsverfahren nach Anspruch 15 oder 16, **dadurch gekennzeichnet, dass** es sich bei den durch Immobilisierung an einer Kultivierungsstruktur kultivierten Miroorganismen um Mikroalgen handelt.

18. Verwendung eines anionischen Tensids bei der Kultivierung phototropher Mikroorganismen, **dadurch gekennzeichnet, dass** das Tensid der zur Kultivierung verwendeten und mit den Mikroorganismen inokulierten oder zu inokulierenden Nährlösung beigegeben wird, um einer mit der Nährlösung benetzten, als Immobilisierungsfläche dienenden Oberfläche einer Kultivierungsstruktur aus einem überwiegend hydrophoben Material durch eine mit der Benetzung einhergehende Veränderung ihrer Oberflächenstruktur einen hydrophilen Charakter zu verleihen und/oder um eine Kontamination der Lösung mit Fremdorganismen, wie Pilzen oder Bakterien zu verhindern.

## Claims

1. A cultivation structure for use in the cultivation of phototrophic microorganisms, in the form of a section of a fabric-like material web, wherein during cultivation at least one of the two large surfaces of the cultivation structure forms an immobilisation surface for microorganisms distributed in a nutrient solution over the respective surface, **characterised in that** the cultivation structure consists of an at least predominantly hydrophobic material but its immobilisation surface or surfaces exhibit a permanently hydrophilic structure as a result of an at least one-time temporary wetting with a wetting agent.

2. The cultivation structure according to claim 1, **characterised in that** this consists of a fabric, of which fibres of at least one hydrophobic polymer are a component.

3. The cultivation structure according to claim 2, **characterised in that** the fabric consists of a fibre mix with fibres of one or more hydrophobic polymers and natural fibres.

4. The cultivation structure according to one of claims 1 to 3, **characterised in that** the cultivation structure is translucent.

5. The cultivation structure according to one of claims 1 to 3, **characterised in that** the cultivation structure possesses at least one immobilisation surface which scatters incident light.

6. The cultivation structure according to one of claims 1 to 3, **characterised in that** the cultivation structure is designed to be light-conducting, wherein light impinging on at least one immobilisation surface is at least partially conducted into the interior of the material.

7. A method for the production of a cultivation structure for the cultivation of phototrophic microorganisms, in which an immobilisation surface is produced on at least one of the large surfaces of a section of a fabric-like material web for microorganisms distributed over the respective surface in a nutrient solution for the purpose of their cultivation, **characterised in that** a material web comprising an at least predominantly hydrophobic material is used for the production of the cultivation structure and
a.) the surface or surfaces of the respective material web provided to form an immobilisation surface is or are wetted with a solution which contains at least one surfactant in a concentration of between 10 ppm and 50 ppm,
b.) at least one section is made up in accordance with the geometry provided for the cultivation structure,
wherein the two above-mentioned steps a.) and b.) can also be carried out in reverse order.

8. The method according to claim 7, **characterised in that** for wetting the surface or surfaces of the hydrophobic material web a solution is used which contains at least one surfactant in a concentration of about 20 ppm.

9. The method according to claim 7 or 8, wherein after the wetting of the or of both of the large surfaces of the material web with the surfactant-containing solution at least one section is made up in accordance with the geometry of the cultivation structure to be formed, **characterised in that** the production of the immobilisation surface or surfaces takes place in the course of the production of the material web by spraying the material web with at least one surfactant.

10. The method according to claim 7 or 8, wherein after the wetting of the or of both of the large surfaces of the material web with the surfactant-containing solution at least one section is made up in accordance with the geometry of the cultivation structure to be formed, **characterised in that** the production of the immobilisation surface or surfaces takes place in the course of the production of the material web by applying at least one surfactant in a dipping process, in which the sections of the material web to be wetted in each case are dipped into the surfactant-containing solution.

11. The method according to claim 7 or 8, wherein an appropriate section for the cultivation structure is made up in accordance with the geometry cultivation structure starting from the material web of the hydrophobic material before the wetting of the or of both of the large surfaces of the material web with the surfactant-containing solution, **characterised in that** the wetting with the surfactant-containing solution takes place while the cultivation structure is being used for cultivation, wherein the at least one surfactant is added to a solution already inoculated or to be inoculated with the phototrophic microorganisms and to be enriched with nutrients for their cultivation and to be applied on to the cultivation structure for the purpose of immobilisation of the microorganisms.

12. The method according to claim 11, **characterised in that** the section(s) of a material web of hydrophobic material acting as a cultivation structure for the cultivation of microorganisms are wetted intensively with the surfactant-containing solution to form at least one immobilisation surface during the commissioning of the cultivation device or while it is first being brought into use as a cultivation structure, to which end an aqueous solution to be inoculated with the microorganisms is passed through the cultivation device in a repeated cycle at a reduced throughput compared with the actual cultivation operation, initially without a wetting of the cultivation structures, during which the surfactant or surfactants are metered into this stepwise and, after mixing thoroughly with the surfactant or surfactants with the addition of nutrients and stepwise increase of the throughput of the nutrient solution, the solution is discharged on to the cultivation structures which are then included in the cycle.

13. The method according to one of claims 7 to 12, **characterised in that** an anionic surfactant is used for wetting the surfaces of the cultivation structure acting as immobilisation surfaces.

14. The method according to claim 13, **characterised in that** for the wetting, linear alkyl benzenesulfonate is used for the wetting.

15. A cultivation method for phototrophic microorganisms, according to which the microorganisms are immobilised on at least one substantially vertically disposed cultivation structure comprising an at least predominantly hydrophobic material, wherein an aqueous nutrient solution to be inoculated with the microorganisms is discharged above the at least one cultivation structure and the microorganisms immobilised on the at least one cultivation structure after inoculation are cultivated with the supply of natural or artificial light and gaseous nutrients, **characterised in that** the nutrient solution discharged above the at least one cultivation structure contains at least one surfactant, wherein the concentration of the at least one surfactant is between 10 ppm and 50 ppm the first time the circulated nutrient solution is discharged.

16. A cultivation method for phototrophic microorganisms using at least one cultivation structure according to claim 1, which was produced by the method according to one of claims 7 to 14, wherein a nutrient solution with the microorganisms contained therein is discharged above the at least one cultivation structure, **characterised in that** surfactant portions which are washed back down from the immobilisation surface or surfaces or are contained in the nutrient solution as excess surfactant portions remain in the nutrient solution for the duration of the cultivation process.

17. The cultivation method according to claim 15 or 16, **characterised in that** the microorganisms cultivated by immobilisation on a cultivation structure are microalgae.

18. Use of an anionic surfactant in the cultivation of phototrophic microorganisms, **characterised in that** the surfactant is added to the nutrient solution used for the cultivation and inoculated or to be inoculated with the microorganisms to impart a hydrophilic nature to a surface of a cultivation structure comprising a predominantly hydrophobic material wetted with the nutrient solution acting as an immobilisation surface by means of a change to its surface structure associated with the wetting, and/or to prevent a contamination of the solution with foreign organisms, such as fungi or bacteria.

## Revendications

1. Structure de culture pour utilisation lors de la culture de microorganismes phototrophes, sous forme d'un segment d'une bande de matériau de type tissu, dans laquelle au moins l'une des deux surfaces d'aire élevée de la structure de culture forme lors de la culture une surface d'immobilisation pour des microorganismes répartis dans une solution nutritive sur la surface considérée, **caractérisée en ce que** la structure de culture est constituée d'un matériau au moins essentiellement hydrophobe, mais que sa ou ses surfaces d'immobilisation, en raison d'un mouillage au moins une fois temporaire avec un mouillant, présentent une structure hydrophile permanente.

2. Structure de culture selon la revendication 1, **caractérisée en ce qu'**elle est constituée d'un tissu, dont un constituant est formé de fibres d'au moins un polymère hydrophobe.

3. Structure de culture selon la revendication 2, **caractérisée en ce que** le tissu est constitué d'un mélange de fibres, comportant des fibres d'un ou plusieurs polymères hydrophobes et des fibres naturelles.

4. Structure de culture selon l'une des revendications 1 à 3, **caractérisée en ce que** la structure de culture est transparente.

5. Structure de culture selon l'une des revendications 1 à 3, **caractérisée en ce que** la structure de culture possède au moins une surface d'immobilisation diffusant la lumière incidente.

6. Structure de culture selon l'une des revendications 1 à 3, **caractérisée en ce que** la structure de culture a une configuration photoconductrice, la lumière tombant au moins sur une surface d'immobilisation étant au moins partiellement dirigée vers l'intérieur du matériau.

7. Procédé de production d'une structure de culture pour la culture de microorganismes phototrophes, dans lequel, sur au moins l'une des surfaces d'aire élevée d'un segment d'une bande de matériau de type tissu, une surface d'immobilisation est produite pour des microorganismes répartis dans une solution nutritive pour leur culture sur la surface considérée, **caractérisé en ce que**, pour fabriquer la structure de culture, on utilise une bande de matériau en un matériau au moins essentiellement hydrophobe, et
a) on mouille avec une solution qui contient au moins un tensioactif à une concentration comprise entre 10 et 50 ppm la ou les surfaces de la bande de matériau considérée, prévues pour réaliser une surface d'immobilisation,
b) on confectionne au moins un segment correspondant à la géométrie prévue pour la structure de culture, dans lequel les deux étapes a) et b) mentionnées ci-dessus peuvent aussi être mises en oeuvre dans l'ordre inverse.

8. Procédé selon la revendication 7, **caractérisé en ce que**, pour mouiller la ou les surfaces de la bande de matériau hydrophobe, on utilise une solution qui contient au moins un tensioactif à une concentration d'environ 20 ppm.

9. Procédé selon la revendication 7 ou 8, dans lequel, après le mouillage de la ou des deux surfaces d'aire élevée de la bande de matériau avec une solution contenant un tensioactif, on confectionne au moins un segment correspondant à la géométrie de la structure de culture à former, **caractérisé en ce que** la production de la ou des surfaces d'immobilisation a lieu au cours de la fabrication de la bande de matériau, par pulvérisation d'au moins un tensioactif sur la bande de matériau.

10. Procédé selon la revendication 7 ou 8, dans lequel, après le mouillage de la ou des deux surfaces d'aire élevée de la bande de matériau avec la solution contenant un tensioactif, on confectionne au moins un segment correspondant à la géométrie de la structure de culture à former, **caractérisé en ce que** la production de la ou des surfaces d'immobilisation a lieu au cours de la fabrication de la bande de matériau par application d'au moins un tensioactif dans un procédé au trempé, dans lequel on immerge dans la solution contenant un tensioactif chacun des segments à mouiller de la bande de matériau.

11. Procédé selon la revendication 7 ou 8, dans lequel on confectionne un segment correspondant pour la structure de culture, en partant de la bande de matériau du matériau hydrophobe avant le mouillage de la ou des surfaces d'aire élevée de la bande de matériau avec une solution contenant un tensioactif, d'une manière correspondant à la géométrie de la structure de culture, **caractérisé en ce que** le mouillage avec la solution contenant un tensioactif a lieu lors de l'utilisation de la structure de culture pour la culture, le ou les tensioactifs étant ajoutés à une solution à laquelle les microorganismes phototrophes ont déjà été inoculés ou doivent être inoculés, et devant être enrichie de nutriments pour la culture, et devant être appliquée sur la structure de culture à des fins d'immobilisation des microorganismes.

12. Procédé selon la revendication 11, **caractérisé en ce que** le ou les segments, servant de structure de culture pour la culture de microorganismes, d'une bande de matériau d'un matériau hydrophobe, sont, pour former au moins une surface d'immobilisation lors de la mise en service du dispositif de culture ou de la première utilisation en tant que structure de culture, mouillés d'une manière intensive avec la solution contenant un tensioactif, ce pour quoi une solution aqueuse, à laquelle les microorganismes doivent être inoculés, est envoyée, à un débit réduit par rapport à celui de l'opération de culture proprement dite, et d'abord sans un mouillage des structures de culture en circuit fermé, à travers le dispositif de culture, on ajoute alors par étapes le ou les tensioactifs, et la solution, après mélange intime avec le ou les tensioactifs, est, après addition de nutriments et augmentation pas à pas du débit de la solution nutritive, répandue sur les structures de culture qui sont alors incorporées dans le circuit fermé.

13. Procédé selon l'une des revendications 7 à 12, **caractérisé en ce que**, pour mouiller les surfaces de la structure de culture servant de surfaces d'immobilisation, on utilise un tensioactif anionique.

14. Procédé selon la revendication 13, **caractérisé en ce que**, pour le mouillage, on utilise un (alkyle linéaire)benzènesulfonate linéaire.

15. Procédé de culture de microorganismes phototrophes, d'après lequel des microorganismes sont immobilisés sur au moins une structure de culture disposée d'une manière essentiellement verticale, constituée d'un matériau au moins essentiellement hydrophobe, dans lequel, au-dessus de la ou des structures de culture, on répand une solution nutritive aqueuse à laquelle doivent être inoculés des microorganismes, et on cultive les microorganismes, immobilisés après l'inoculation sur la ou les structures de culture, avec apport d'une lumière naturelle ou artificielle, ainsi que de nutriments gazeux, **caractérisé en ce que** la solution nutritive répandue au-dessus de la ou des structures de culture contient au moins un tensioactif, la concentration du ou des tensioactifs, lors de la première application de la solution nutritive circulant en circuit fermé, étant comprise entre 10 et 50 ppm.

16. Procédé de culture de microorganismes phototrophes par utilisation d'au moins une structure de culture selon la revendication 1, qui avait été fabriquée par le procédé selon l'une des revendications 7 à 14, dans lequel une solution nutritive contenant les microorganismes est répandue au-dessus de la ou des structures de culture, **caractérisé en ce que** les fractions du tensioactif qui sont de nouveau lavées de haut en bas par la ou les surfaces d'immobilisation, ou qui sont contenues dans la solution nutritive en tant que fractions de tensioactif en excès, restent dans la solution nutritive pendant toute la durée de l'opération de culture.

17. Procédé de culture selon la revendication 15 ou 16, **caractérisé en ce que**, pour ce qui concerne les microorganismes cultivés par immobilisation sur une structure de culture, il s'agit de micro-algues.

18. Utilisation d'un tensioactif anionique lors de la culture de microorganismes phototrophes, **caractérisée en ce que** le tensioactif est ajouté à la solution nutritive utilisée pour la culture et à laquelle les microorganismes sont inoculés ou doivent être inoculés, pour conférer un caractère hydrophile à une surface, mouillée par la solution nutritive, servant de surface d'immobilisation, d'une structure de culture en un matériau essentiellement hydrophobe, grâce à une modification, consécutive au mouillage, de sa structure superficielle, et/ou pour empêcher une contamination de la solution par des organismes étrangers tels que des champignons ou des bactéries.
